# EUROPEAN PATENT APPLICATION

(11) **EP 4 563 134 A1**
(43) Date of publication of application: **04.06.2025**
(21) Application number: 23307110.9
(22) Date of filing: 30.11.2023
(51) Int. Cl.: A61H 3/00, A61B 5/00, A61H 1/00, A61B 5/11, A61B 5/16, A61B 5/024, A61B 5/01

(54) **SYSTEM FOR PROVIDING MOTION ASSISTANCE**

(71) Applicant: Essilor International, 94220 Charenton-Le-Pont (FR)
(72) Inventor: GIRAUDET, Guillaume, 45000 ORLEANS (FR); SAHLER, Jean, 92160 ANTONY (FR); AMIR, Bruno, 94100 SAINT MAUR DES FOSSES (FR); GIL, Paul, 80000 AMIENS (FR)
(74) Representative: Plasseraud IP

(57) **Abstract**

A system (3) for providing motion assistance to a user, comprising at least one sensor (5) for measuring values of at least one motion parameter and/or at least one stress parameter, the system comprising at least one stimulus generating module (7), configured to generate at least one stimulus for the user.

The system is configured for:
- obtaining (OBT) through the sensor (5) a value of the at least one motion parameter and/or a value of the at least one stress parameter,
- comparing (COMP) the value of the motion parameter to a motion reference value and/or the value of the stress parameter to a stress reference value and determining a motion behavior of the user, and
- generating, according to the determined motion behavior, the at least one stimulus through the at least one stimulus generating module (7), to assist a motion of the user.

## Description

### Field of the invention

The invention relates to the field of wearable electronic device for providing assistance to a user, in particular motion assistance to users suffering from Parkinson's disease.

More specifically, the invention relates to a system for providing motion assistance to a user. The invention also relates to a head-mountable device, a pair of eyeglasses and a method for providing motion assistance to a user.

### Background of the invention

An issue associated with Parkinson's disease is difficulty for the patients to walk normally. One of the min problems at the source is the inability to maintain an adequate stride length, compensating instead with multiple short steps, which are less efficient and less stable. As the disease eventually progresses, difficulty can be observed in initiating a walking motion, in maintaining the walking motion, in taking turns, or an inability to chain steps efficiently. These difficulties can lead to a freezing of gait and the patient falling due to the irregular and uncontrolled walking pattern.

This freezing of gait is one of the most debilitating and frequent symptoms of Parkinson's disease, and so far, cannot be improved by medical or surgical treatment, requiring alternative palliative options.

There have been attempts to alleviate at least partly the freezing of gait issues, in order to prevent the associated inconvenience and risks.

For instance, it has been shown that patients suffering from Parkinson's disease respond well to rhythmic auditory or verbal stimuli, which help maintaining a regular walking pattern. However, delivering such an auditory assistance to the patient is difficult in real-world conditions. For example, a tried solution is the use of earplugs placed in the patient's ears delivering the rhythmic stimulus. A problem encountered with this solution is the selection of the correct level of sound, which can be either too loud and hamper the patient's hearing, or too quiet, and become inefficient. This sound level further depends on the outside sound level, which may vary depending on the place and situation, as well as on the audition of the patient. Furthermore, the attention required from the patient to the auditory stimuli may prevent the patient from paying sufficient attention to the surroundings, which may lead to dangerous situations.

Another type of envisioned sensory walking assistance is based on providing visual stimuli to the patient to improve the patient's gait. However, such visual stimuli are even more complicated to deliver in a practical manner in a real-world environment.

The most efficient type of visual stimulus is the presence of ground markings, such as bands on the grounds, with a predetermined spacing allowing the patient to adjust their stride length. While efficient in a specifically prepared environment, this type of assistance cannot be extended to every kind of environment met by the patients.

Another type of visual cues is use of a head-mountable device equipped with a light source arranged to project similar markings of the ground in front of the user. However, this type of device is not as effective as actual ground markings, and the light source may cause other types of inconvenience in real life, especially a laser source.

Finally, active devices of this type can consume large amounts of energy and generate heat, making their prolonged use complicated.

There is thus a need for a system to provide a motion assistance to users, in particular a walking assistance, with minimal impact inconvenience on their daily life and usual activities.

### Summary of the invention

To this end, the invention relates to a system for providing motion assistance to a user, the system comprising at least one sensor for measuring values of at least one motion parameter, representative of a movement of the user, and/or at least one stress parameter, representative of a stress level of the user,
the system comprising at least one stimulus generating module, configured to generate at least one stimulus for the user, the system being configured for:
- obtaining through the at least one sensor a value of the at least one motion parameter and/or a value of the at least one stress parameter,
- comparing the value of the at least one motion parameter to at least one motion reference value and/or the value of the at least one stress parameter to at least one stress reference value and determining a motion behavior of the user, and
- generating, according to the determined motion behavior, the at least one stimulus through the at least one stimulus generating module, to assist a motion of the user.

Such a system allows to monitor the occurrence of a need for assistance of the user and to provide a walking assistance to the user by generating sensor stimuli only when a behavior indicative of a need for assistance is determined, reducing the overall energy expenditure and heating as well as preventing the generation of unnecessary stimuli.

The at least one motion parameter may comprise at least one of:
- a frequency of steps taken by the user,
- a length of the steps taken by the user,
- an amplitude of tremors experienced by the user, and/or
- a Fourier transform of a signal representative of an acceleration of the user.

This feature allows to monitor specific parameters that characterize a motion of the user to detect variations indicating a need for assistance due to a motion associated difficulty.

The at least one stress parameter may comprise at least one of:
- a heart rate of the user,
- a variability of the heart rate of the user,
- an intensity of a cardiac activity of the user,
- an increase of a corporal temperature of the user,
- a spontaneous blink rate of the user,
- a sweating intensity of the user, and/or
- a level of an electrodermal activity of the user.

This feature allows a monitoring of a parameter that is representative of a stress level of the user, linking variations of this parameter to a high stress situation, which allows to discriminate between stressful situations linked to a motion behavior indicative of an associated difficulty and other unrelated stressful situations.

The system may further comprise a postural sensor configured to obtain values of at least one postural parameter, representative of a posture of the user, the system being configured to detect a standing position of the user from the values of the at least one postural parameter, the system being configured to generate the at least one stimulus only when a standing position is detected.

This feature allows to discriminate quickly between high stress levels and motions indicative of a difficult linked to walking, that only occur when standing, and other type of situations that may cause an elevated stress level and/or specific associated motions of the user.

The system may further comprise at least one environmental sensor configured to obtain values of at least one environmental parameter representative of a close environment of the user, the system being further configured to compare the obtained values of the environmental parameter to reference values for detecting a difficult environment, and to generate the at least one stimulus when a difficult environment is detected.

This feature allows to detect external conditions that are likely to cause an elevated stress of the user and may cause difficulties to walk, in order to pre-emptively assist the user.

The at least one environmental sensor may comprise at least one of a barometric pressure sensor, a camera, a radiofrequency sensor, an infrared sensor, a time-of-flight sensor, a light imaging detection and a ranging sensor.

This feature allows the monitoring of an environment of the user by implementing continuous measurements of relevant environmental parameters.

The at least one stimulus generating module comprises a laser source, configured for projecting lines of light on a floor in front of the user to assist the user at adjusting a length of steps.

The at least one stimulus generating module may comprise a display interface, preferably integrated in at least one optical lens of the system, the display interface being configured to generate reference images in the field of vision of the user to assist the user at adjusting a length of steps.

This feature allows to generate visual stimuli directly in the field of vision of the user, without the inconvenience associated with a light source projecting light on the ground.

The system may be further configured to display each reference image in a fixed position relative to the environment of the user, the system being configured to displace the image in the field of vision of the user when the user moves.

This feature allows the reference images to stand in place while the user walks, which improves the assistance provided to the user.

The system may further comprise a device for adjusting a transmittance of the at least one optical lens, the system being configured to adjust the transmittance of the at least one optical lens at least in an area where the reference images are generated, to improve a visibility of the reference images by the user.

This feature allows to improve the visibility of the reference images for the user, in particular in a bright environment.

The at least one stimulus generating module may comprise an acoustic device configured to generate a sound at a controlled frequency and a controlled intensity in at least one ear of the user and/or a haptic device, configured to generate haptic stimuli at a controlled frequency and a controlled intensity through at least one body part of the user, for example in the vicinity of a bone, to assist the user at adjusting a frequency of steps.

This feature allows for generating an acoustic stimulus, transmitted through the air and/or through a body part of the user, and assisting the user in maintaining an appropriate step frequency.

The system further may comprise an ambient sound sensor configured to detect an intensity and/or a frequency profile of the ambient sound in an environment of the user, the system being configured to adjust an intensity and/or a frequency of the generated acoustic stimuli to improve a perceivability of the acoustic stimuli by the user.

This feature allows to adjust the intensity of the acoustic stimuli to be perceptible over the ambient noise but not excessively loud, and/or to adjust the main frequency of the acoustic stimuli to be apart from a main frequency of the background noise to improve perceptibility.

The invention also relates to a head-mountable device comprising a frame and a system for providing motion assistance to a user, the system comprising at least one sensor for measuring values of at least one motion parameter, representative of a movement of the user, and/or at least one stress parameter, representative of a stress level of the user,
the system comprising at least one stimulus generating module, configured to generate at least one stimulus for the user, the system being configured for:
- obtaining through the at least one sensor a value of the at least one motion parameter and/or a value of the at least one stress parameter,
- comparing the value of the motion parameter to at least one motion reference value and/or the value of the at least one stress parameter to at least one stress reference value and determining a motion behavior of the user, and
- generating, according to the determined motion behavior, the at least one stimulus through the at least one stimulus generating module, to assist a motion of the user.

The invention further relates to eyeglasses comprising a system for providing motion assistance to a user, the system comprising at least one sensor for measuring values of at least one motion parameter, representative of a movement of the user, and/or at least one stress parameter, representative of a stress level of the user,
the system comprising at least one stimulus generating module, configured to generate at least one stimulus for the user, the system being configured for:
- obtaining through the at least one sensor a value of the at least one motion parameter and/or a value of the at least one stress parameter,
- comparing the value of the motion parameter to at least one motion reference value and/or the value of the at least one stress parameter to at least one stress reference value and determining a motion behavior of the user, and
- generating, according to the determined motion behavior, the at least one stimulus through the at least one stimulus generating module, to assist a motion of the user.

The invention also relates to a computer-implemented method for providing motion assistance to a user, the method comprising:
- obtaining through at least one sensor a value of at least one motion parameter and/or a value of at least one stress parameter,
- comparing the value of the at least one motion parameter to at least one motion reference value and/or the value of the at least one stress parameter to at least one stress reference value and determining a motion behavior of the user, and
- commanding, according to the determined motion behavior, at least one stimulus generating module to generate at least one stimulus, to assist a motion of the user.

### Brief description of the drawings

Figure 1 is a perspective view of a head-mountable device comprising a system after the invention,
Figure 2 is a perspective view of a user wearing a head-mountable device after the invention providing motion assistance,
Figure 3 is a rear view of another head-mountable device comprising a system after the invention and providing motion assistance to a user, and
Figure 4 is a schematic representation of steps of a method for providing motion assistance after the invention.

### Detailed description of embodiments

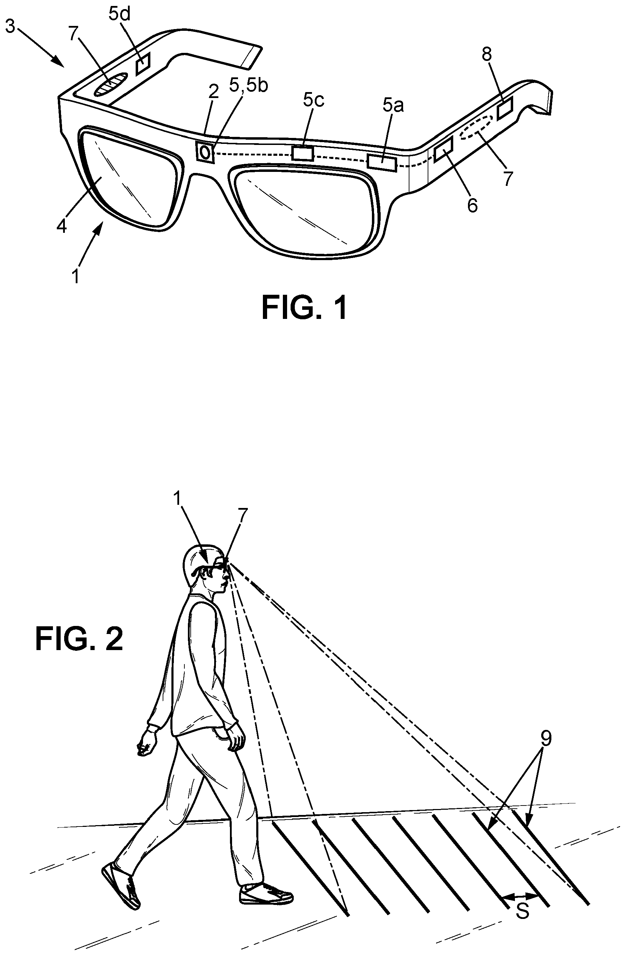

A first implementation of the invention is described below, in reference to figure 1. In this implementation, a system 3 for providing motion assistance according to the invention is mountable on a head-mountable device 1 of the type commonly designated as smart glasses, shown on figure 1.

Such a head-mountable device 1 typically comprises a rigid frame 2 comprising a single part or several articulated parts, arranged for being put on the face of a user by resting on at least one facial feature such as the nose and/or ears.

The head-mountable device further comprises at least one optical element, such as a pair of lenses 4, that may provide an ophthalmic function, or be plano lenses.

Advantageously, the lenses 4 are equipped with a display interface, configured to display images in the field of vision of the user.

The lenses 4 may also present a controllable optical property, such as a controllable light transmission. For example, the lenses 4 comprise an electrochromic element.

The system 3 comprises at least one sensor 5 for obtaining values of at least one motion parameter, representative of a position and/or of movements of the user of the head-mountable device 1, and/or at least one sensor 5 for obtaining values of at least one stress parameter, representative of a stress level of the user.

The system 3 also comprises a calculation module 6, comprising an electronic circuit comprising at least one processor, for executing programs, and a memory for storing data and instructions for the execution of said programs.

The calculation module 6 is connected to each sensor 5 on the device 1, ether by direct wire or through a wireless interface, for piloting the sensor 5 and for analyzing and saving the obtained information.

The system 3 further comprises at least one stimulus generating module 7, configured to generate at least one stimulus destined to the user to assist a motion of the user, notably to assist the user walking.

The at least one stimulus is preferably a sensory stimulus, which is a stimulus destined to be perceived by the used through at least one sense of the user. For example, each of the at least one stimulus generating module 7 is configured to generate visual and/or acoustic stimuli for the user. The visual stimuli are perceived by sight and comprise images in the user's field of vision. The acoustic stimuli may be perceived by hearing, and comprise sound directed toward the user's ears, and/or be perceived by touch, and comprise haptic stimuli, which are acoustic vibrations generated in at least one body part of the user.

Each of the at least one sensor 5 and the at least one stimulus generating module 7 may be integrated to the frame 2 of the head-mountable device 1, mountable to the frame 2 in a detachable manner, or mountable on an external device that may be worn by the user.

The at least one sensor 5 for obtaining values of at least one motion parameter may comprise at least one inertial motion sensor 5a (also known as an IMU, for Inertial Motor Unit), which measures linear acceleration (i.e. acceleration in a straight line), a gyroscopic sensor, which measures angular movements and positions, a magnetic sensor, for measuring orientations relative to a reference direction, as well as any sensor that measures information that can be related to movement or position, to obtain the values of the motion parameter.

Such a sensor may allow the system 3 to obtain values of a frequency of steps taken by the user, by analyzing the measured acceleration and frequency of variation thereof, for example, as well as a length of steps taken by the user.

Furthermore, this type of sensor 5 may also be configured to measure an amplitude of tremors experienced by the user, for example as a consequence of a tremor crisis caused by Parkinson's disease and use this amplitude of tremors as one of the at least one the motion parameter.

In particular, the system 3 may be configured to calculate a Fourier transform of a signal, or function, representative of the acceleration of the user, to obtain information relative to the frequency of steps, for example, and/or the amplitude of tremors, by measuring an amplitude of the frequency band corresponding to the tremors in the Fourier transform.

Such an inertial motion sensor 5a may also be used as a postural sensor, configured to obtain values of at least one postural parameter, representative of a posture of the user. In this case, the system 3 is configured to detect a standing position of the user from the values of the at least one postural parameter, and preferably to differentiate between a standing position and a sitting or lying position of the user.

The at least one sensor 5 may also include at least one camera 5b mountable on the device 1 and associated with appropriate image treatment algorithm, for example run by the calculation module, to derive the values of the motion parameter therefrom.

The camera 5b and the image treatment algorithm may also be configured to act as a postural sensor, to obtain values of the at least one postural parameter as mentioned above.

The camera 5b may be arranged facing a front of the user and to capture image of the environment of the user.

The camera 5b may also be used as an environmental sensor, in addition or instead of a positional sensor, to obtain information relative to an environment of the user and derive values of at least one environmental parameter.

The at least one sensor 5 may also comprise one or more other environmental sensors 5c, in addition or instead of the camera 5b, configured to obtain values of the at least one environmental parameter.

The environmental parameter is representative of a close environment of the user. For example, the environmental parameter may be indicative of one or more obstacles close to the user, such as a distance between the user and the nearest obstacle. For example, the at least one environmental sensor 5c may comprise a radiofrequency sensor, an infrared sensor, a time-of-flight sensor, a light imaging detection and a ranging sensor.

Alternatively, the environmental parameter may be representative of the situation or activity of the user, such as whether the user is climbing a flight of stairs. The at least one environmental sensor may comprise at least one of a barometric pressure sensor, able to measure a variation of altitude of the user.

The at least one sensor 5 may also comprise at least one physiological sensor 5d, configured to measure values of at least one stress parameter representative of a physiological stress level experienced by the user.

The physiological sensor 5d is for example a sensor for measuring a heart rate and/or a blood pressure of the user, positioned for example against a temple of the user. In this example, the system 3 is configured to obtain values of the stress parameter therefrom, for example values of a heart rate, a variability of the heart rate, or an intensity of a cardiac activity of the user, with higher values of heart rate, cardiac intensity, or an increase in variability of the heart rate being representative of a higher stress level of the user.

The physiological sensor 5d may also be configured to measure at least one parameter of the skin of the user, representative of a physiological stress level of the user. The measured skin parameter is for example a skin temperature, an electrodermal activity of the user, and/or an amount of sweat produced by the skin of the user. The physiological sensor 5d comprises, for example, a capacitive probe and/or a thermocouple. The measured physiological parameter may be inferred from the measured values, with an elevation of sin temperature, electrodermal activity and/or sweating intensity being representative of a higher stress level of the user. The physiological sensor 5d may also comprise a camera arranged to acquire images of the user's eyes, combined with a suitable image treatment algorithm to calculate a spontaneous blink rate of the user. The spontaneous blink rate may be used as a stress parameter, with high values of the spontaneous blink rate being representative of a higher level of stress of the user.

The system 3 is configured to compare the obtained values of the at least one motion parameters to at least one motion reference value and/or the obtained values of the at least one stress parameter to at least one stress reference value to determine a motion behavior of the user, and notably detect a motion behavior indicative of a difficulty of the user and thus detect or predict a need for assistance.

The motion reference values and stress reference values are for example threshold values of the corresponding parameter that, when crossed, indicate that motion behavior of the user is not a regular motion behavior, and that the user is facing a motion difficulty and requires assistance.

For example, an increase of the step frequency and/or a reduction in step length is often a sign of a starting freezing of gait and may be detected by the system 3 to provide assistance to alleviate the freezing of gait.

An increase in the intensity of Parkinson tremors may also be detected through this comparison and trigger an assistance to the user.

Furthermore, elevated levels of stress can also be correlated with an incoming freezing of gait, and the comparison of the obtained values of the stress parameter with the reference values allows the detection of the freezing of gait and the assistance.

The reference values are for example stored in and taken from the memory of the calculation module of the system 3. Advantageously, the reference values are adjusted to the user and to the user's specific preferences and characteristics.

Values of several motion parameters and/or stress parameters may be obtained and compared to corresponding reference values simultaneously to enhance the determination of the motion behavior and eventual detection of a difficulty, for example by applying specific weights to each parameter and by considering the margin by which the obtained values differ from the corresponding reference values.

Furthermore, the system 3 may be configured to take into accounts obtained values of the at least one positional parameter and/or the at least one environmental parameter in detecting a need for assistance.

For example, if the obtained values of the positional parameter indicate that the user is sitting or lying, providing a walking assistance is not necessary and no corresponding stimuli will be generated. Instead, if the user is in a standing position, a walking assistance may be necessary, and will be implemented if such a need is detected.

Furthermore, the obtained values of the environmental parameter may indicate a situation more likely to require assistance, such as moving in a complex environment with several obstacles or moving up or down stairs. In such a case, the system is configured to be more likely to generate stimuli to provide assistance, for example by using lower thresholds for the reference values of the motion and/or stress parameters.

The at least one stimulus generating module 7 may comprise at least one microphone configured to generate sound towards or inside the user's ears. The sound is emitted with a controlled frequency and a controlled intensity, or with a controlled frequency profile comprising several frequency or frequency bands, and a controlled intensity profile over the frequency profile.

In an embodiment, the microphones are located in earplugs placed in the user's ears and communicating with the calculation module through a wireless interface.

In another embodiment, the microphones are mounted on the frame 2, for example on the branches, and are directional speakers placed to emit sound toward the ears of the user. The acoustic stimuli generated may be a rhythmic sound, such as for example pulses emitted at a single stable frequency, to help maintain an adequate stride frequency of the user. Alternatively, the sound may be music, selected so that a dominant frequency in the music matches an adequate step frequency of the user.

Advantageously, the system 3 comprises at least one ambient sound sensor 8 configured to detect an intensity and/or a frequency profile of the ambient sound in an environment of the user. The system 3 is then configured to adjust an intensity and/or a frequency of the generated acoustic stimuli to improve a perceivability of the acoustic stimuli by the user.

For example, in an environment with a high ambient sound level, the system 3 is configured to increase an intensity of the acoustic stimuli to make them perceptible over the background noise.

In the case of a low intensity ambient sound, the system 3 would reduce the intensity of the stimuli to maintain the comfort of the user.

In the case of an environment with a predominant frequency, such as high-pitch or low-pitch dominant background noise, the system 3 is configured to shift the frequency of the sound in the acoustic stimuli away from the dominant frequency of the environment, to improve perceptibility.

Advantageously, the system 3 is also configured to adapt the frequency of the sound to the hearing of the user, taking into account hearing difficulties at certain frequencies to select the main sound frequency as a perceptible frequency.

In another embodiment, the stimulus generating module 7 may comprise at least one haptic device, as shown on figure 1, comprising for example at least one vibrating surface placed in contact with the skin of the user, preferably in the vicinity of at least one bone of the user. In this embodiment, the acoustic stimuli may be generated as haptic signals. The haptic device may be located on the frame 2, for example in the branches, facing the temples of the user.

The haptic pulses may be generated with an intensity and a frequency selected to obtain a good conduction of the signal through the bones of the user, to make the stimuli easy to perceive without discomfort. As the stimuli are not designed to convey any meaning, the narrow band of adapted frequencies is not an issue.

As represented on figure 2, the at least one stimulus generating module 7 may comprise a light source, for example a laser source, configured to display visual stimuli directly on the environment in front of the user, such as parallel light lines with a spacing S separating the lines sensibly equal to an adequate stride length. For example, the light source is equipped with a controlled collimating device and piloted by the calculation module 6 to display the lines 9 as a visual stimulus in case of a need of assistance.

Advantageously, the laser source may be mounted on a gyro-stabilized mount on the frame 2, in order to compensate for steps induced movements and display the lines 9 in a stable arrangement.

Alternatively, or in addition, the laser source may be piloted by the system 3 in relation with movement information obtained through the inertial motor unit 5a, in order to compensate for the head movement and body movement of the user and display the reference lines 9 in fixed positions relative to the ground.

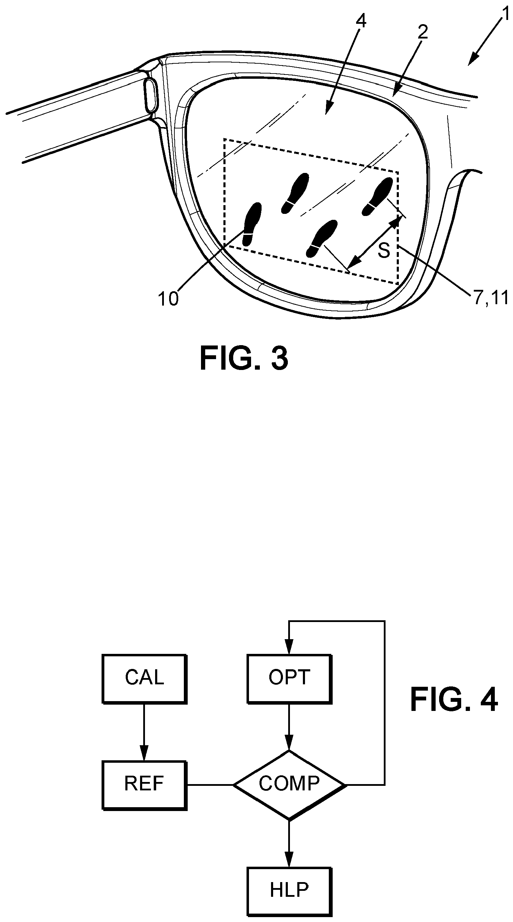

In another embodiment, represented on figure 3, the stimulus generating module 7 may be configured to display the images on the lenses 4 of the head-mountable device 1.

For example, the visual stimuli are images 10 displayed in a process of augmented reality, superimposed on the field of vision of the user by smart lenses 4 comprising a display area 11. The images may be for example footprints displayed on the ground, in the lower part of the lens 4, and spaced adequately with a spacing S configured to help the user maintain an adequate stride length.

Advantageously, the system 3 may be configured to detect features of an image taken in front of the user, for example through the camera 5b, and associate the position of each image with coordinates relative to these features, so that the images are unmoving relative to the environment of the user when the user moves. This type of unmoving reference images is more efficient in assisting the user than moving lines.

Advantageously, the system 3 may be configured to adjust the light transmission of the lenses 4 in an area surrounding the images, for example in the display area 11, in order to improve their visibility by the user. For example, in case of a bright floor, the display area 11 may be darkened to better display the images 10. The variation of the light transmission may be implemented for example by controlling an electrochromic element included in the lenses 4. Advantageously, properties of the images 10 may be adapted to the surface they are displayed on, for example the intensity, color and type of image may be modified to provide additional information. The type of surface may be determined by analyzing images provided by the camera 5a and implementing an image recognition algorithm, for example of the artificial intelligence type.

Advantageously, the stride length S indicated by the visual stimuli can be adjusted to match the user's normal stride length and may be further adjusted in relation to the perceived environment. For example, in an environment comprising many identified obstacles, the target stride length may be reduced.

A computer-implemented method for providing motion assistance to a user, implanted by the system 3 described above, is described below in reference to figure 4.

The method comprises an optional preliminary calibration step CAL, comprising the selection of reference values for the at least one motion parameter and/or stress parameter, from the memory of the calculation module 6, taking into account eventual selected user preferences, and/or obtained values of the at least one motion parameter, stress parameter, environment parameter and/or positional parameter.

This calibration step CAL may be implemented again automatically in case of a detected change in situation or environment of the user, for example to adapt the selected reference values to a new situation or environment.

The selected reference values REF are stored in the memory for use in the method until a change is required.

The method comprises a step of obtaining OBT through at least one sensor 5 a value of at least one motion parameter and/or a value of at least one stress parameter, as described above. The obtained values are for example stored in the memory.

The method then comprises a step of comparing COMP the value of the at least one motion parameter to at least one motion reference value and/or the value of the at least one stress parameter to at least one stress reference value and determining a motion behavior of the user.

The method finally comprises a step of commanding HLP, according to the determined motion behavior, the at least one stimulus generating module 7 to generate at least one stimulus, to assist a motion of the user, as disclosed above.

If the determined motion behavior is normal, and thus no need for assistance is detected, the step of obtaining OBT values of the stress parameter and the motion parameter are iterated to monitor the need for assistance of the user.

## Claims

1. A system (3) for providing motion assistance to a user, the system (3) comprising at least one sensor (5) for measuring values of at least one motion parameter, representative of a movement of the user, and/or at least one stress parameter, representative of a stress level of the user,
the system comprising at least one stimulus generating module (7), configured to generate at least one stimulus for the user, the system being configured for:
- obtaining (OBT) through the at least one sensor (5) a value of the at least one motion parameter and/or a value of the at least one stress parameter,
- comparing (COMP) the value of the at least one motion parameter to at least one motion reference value and/or the value of the at least one stress parameter to at least one stress reference value and determining a motion behavior of the user, and
- generating, according to the determined motion behavior, the at least one stimulus through the at least one stimulus generating module (7), to assist a motion of the user.

2. The system (3) according to claim 1, wherein the at least one motion parameter comprises at least one of:
- a frequency of steps taken by the user,
- a length of the steps taken by the user,
- an amplitude of tremors experienced by the user, and/or
- a Fourier transform of a signal representative of an acceleration of the user.

3. The system (3) according to claim 1 or 2, wherein the at least one stress parameter comprises at least one of:
- a heart rate of the user,
- a variability of the heart rate of the user,
- an intensity of a cardiac activity of the user,
- an increase of a corporal temperature of the user,
- a spontaneous blink rate of the user,
- a sweating intensity of the user, and/or
- a level of an electrodermal activity of the user.

4. The system (3) according to any one of claims 1 to 3, wherein the system (3) further comprises a postural sensor (5a, 5b) configured to obtain values of at least one postural parameter, representative of a posture of the user, the system being configured to detect a standing position of the user from the values of the at least one postural parameter, the system being configured to generate the at least one stimulus only when a standing position is detected.

5. The system (3) according to any one of claims 1 to 4, wherein the system further comprises at least one environmental sensor (5b, 5c) configured to obtain values of at least one environmental parameter representative of a close environment of the user, the system being further configured to compare the obtained values of the environmental parameter to reference values for detecting a difficult environment, and to generate the at least one stimulus when a difficult environment is detected.

6. The system (3) according to claim 5, wherein the at least one environmental sensor (5b, 5c) comprises at least one of a barometric pressure sensor, a camera (5b), a radiofrequency sensor, an infrared sensor, a time-of-flight sensor, a light imaging detection and a ranging sensor.

7. The system (3) according to any one of claims 1 to 6, wherein the at least one stimulus generating module (7) comprises a laser source, configured for projecting lines (9) of light on a floor in front of the user to assist the user at adjusting a length of steps.

8. The system (3) according to any one of claims 1 to 7, wherein the at least one stimulus generating module (7) comprises a display interface (11), preferably integrated in at least one optical lens of the system (4), the display interface (11) being configured to generate reference images (10) in the field of vision of the user to assist the user at adjusting a length of steps.

9. The system (3) according to claim 8, wherein the system (3) is further configured to display each reference image (10) in a fixed position relative to the environment of the user, the system being configured to displace the image (10) in the field of vision of the user when the user moves.

10. The system (3) according to claim 8 or 9, wherein the system further comprises a device for adjusting a transmittance of the at least one optical lens (4), the system (3) being configured to adjust the transmittance of the at least one optical lens (4) at least in an area where the reference images (10) are generated, to improve a visibility of the reference images by the user.

11. The system (3) according to any one of claims 1 to 10, wherein the at least one stimulus generating module (7) comprises an acoustic device configured to generate acoustic stimuli comprising a sound at a controlled frequency and a controlled intensity in at least one ear of the user and/or a haptic device, configured to generate haptic stimuli at a controlled frequency and a controlled intensity through at least one body part of the user, for example in the vicinity of a bone, to assist the user at adjusting a frequency of steps.

12. The system (3) according to claim 11, wherein the system (3) further comprises an ambient sound sensor (8) configured to detect an intensity and/or a frequency profile of the ambient sound in an environment of the user, the system (3) being configured to adjust an intensity and/or a frequency of the generated acoustic stimuli to improve a perceivability of the acoustic stimuli by the user.

13. A head-mountable device (1) comprising a frame (2) and a system (3) for providing motion assistance to a user, the system (3) comprising at least one sensor (5) for measuring values of at least one motion parameter, representative of a movement of the user, and/or at least one stress parameter, representative of a stress level of the user,
the system (3) comprising at least one stimulus generating module (7), configured to generate at least one stimulus for the user, the system (3) being configured for:
- obtaining through the at least one sensor (5) a value of the at least one motion parameter and/or a value of the at least one stress parameter,
- comparing the value of the motion parameter to at least one motion reference value and/or the value of the at least one stress parameter to at least one stress reference value and determining a motion behavior of the user, and
- generating, according to the determined motion behavior, the at least one stimulus through the at least one stimulus generating module (7), to assist a motion of the user.

14. Eyeglasses comprising a system (3) for providing motion assistance to a user, the system (3) comprising at least one sensor (5) for measuring values of at least one motion parameter, representative of a movement of the user, and/or at least one stress parameter, representative of a stress level of the user,
the system (3) comprising at least one stimulus generating module (7), configured to generate at least one stimulus for the user, the system (3) being configured for:
- obtaining through the at least one sensor (5) a value of the at least one motion parameter and/or a value of the at least one stress parameter,
- comparing the value of the motion parameter to at least one motion reference value and/or the value of the stress parameter to at least one stress reference value and determining a motion behavior of the user, and
- generating, according to the determined motion behavior, the at least one stimulus through the at least one stimulus generating module (7), to assist a motion of the user.

15. A computer-implemented method for providing motion assistance to a user, the method comprising:
- obtaining (OBT) through at least one sensor (5) a value of at least one motion parameter and/or a value of at least one stress parameter,
- comparing (COMP) the value of the at least one motion parameter to at least one motion reference value and/or the value of the at least one stress parameter to at least one stress reference value and determining a motion behavior of the user, and
- commanding (HLP), according to the determined motion behavior, at least one stimulus generating module (7) to generate at least one stimulus, to assist a motion of the user.
